# EUROPÄISCHE PATENTSCHRIFT

(11) **EP 1 409 722 B1**
(45) Veröffentlichungstag und Bekanntmachung des Hinweises auf die Patenterteilung: **25.03.2009**
(21) Anmeldenummer: 01994813.2
(22) Anmeldetag: 19.12.2001
(51) Int. Cl.: C12Q 1/68

(54) **VERFAHREN ZUR ANALYSE VON MAKROMOLEKÜLEN**
METHOD FOR ANALYSING MACROMOLECULES
PROCEDE D'ANALYSE DE MACROMOLECULES

(30) Priorität: 30.01.2001 DE 10103954
(43) Veröffentlichungstag der Anmeldung: 21.04.2004
(73) Patentinhaber: Advalytix AG, 85649 Brunnthal (DE)
(72) Erfinder: GAUER, Christoph, 81667 München (DE)
(74) Vertreter: Manitz, Finsterwald & Partner GbR
(86) Internationale Anmeldenummer: PCT/EP2001/015032
(87) Internationale Veröffentlichungsnummer: WO 2002/064821

(56) Entgegenhaltungen:
- EP-A- 1 053 784
- WO-A-94/27719
- WO-A-99/43688
- US-A- 5 653 939
- US-A- 5 674 742
- US-A- 6 010 316
- WANG JOSEPH: "From DNA biosensors to gene chips." NUCLEIC ACIDS RESEARCH, Bd. 28, Nr. 16, 15. August 2000 (2000-08-15), Seiten 3011-3016, XP002221216 ISSN: 0305-1048

## Beschreibung

Die Erfindung betrifft ein Verfahren zur Analyse von Makromolekülen mit Hilfe eines Microarrays.

Eine Methode zur schnellen Analyse von Makromolekülen umfaßt die Aufbringung in einer regelmäßigen Anordnung auf einem sogenannten Microarray. Die Dichten, die heute auf einem solchen Microarray erreicht werden können, betragen z. B. für DNA (Desoxyribonukleinsäure) bis zu ca. 250.000 verschiedene Arten von molekülen pro cm². Eine parallele und schnelle Aufbringung der Moleküle ist z. B. mit sogenannten Pipettierrobotern möglich. In einer solchen Arraygeometrie kann eine Analyse, z. B. eine Fluoreszenzmessung einfach durchgeführt werden ("DNA Microarrays", herausgegeben von M. Schena, Oxford University Press, New York, 1999). Untersuchte Stoffe sind z. B. Antikörper, Antigene, Proteine oder DNA.

In einem solchen Microarray sind verschiedenartige Makromoleküle an verschiedenen Stellen in einer Matrixform angeordnet. Eine Flüssigkeit mit einer anderen Art von Makromolekülen wird über das Microarray gespült, die mit mindestens einer Art von Makromolekülen auf dem Microarray eine spezifische Bindung eingeht. Wird dann die Flüssigkeit wieder von der Oberfläche entfernt, verbleiben nur an den Stellen der spezifischen Bindung die zu untersuchenden Makromoleküle zurück. Mit Hilfe einer ortsaufgelösten Messung, z. B. einer Fluoreszenzmessung, läßt sich feststellen, an welchen Stellen das zu untersuchende Makromolekül vorhanden ist. Aus der bekannten Lage der einzelnen Makromoleküle in der Matrixform des Microarrays kann also festgestellt werden, mit welcher Art von Makromolekülen das zu untersuchende Makromolekül eine spezifische Bindung eingegangen ist.

Dabei muß sichergestellt sein, daß die zu untersuchenden Makromoleküle mit allen Makromolekülen in der Matrixform in Kontakt kommen können. Dazu muß z. B. die gesamte Oberfläche des Substrates, auf dem sich das Microarray befindet, mit der Flüssigkeit mit dem zu untersuchenden Makromolekül überschwemmt werden.

Die Dauer eines entsprechenden Experimentes ist durch die Diffusion bestimmt und kann daher einige Zeit in Anspruch nehmen. Ist z. B. die Konzentration des zu untersuchenden Makromoleküles in der Flüssigkeit nur gering, so kann es sehr lange dauern, bis es seinen spezifischen Bindungspartner auf dem Array gefunden hat. Darüber hinaus hängt die Reaktionskinetik der spezifischen Bindung von vielen Reaktionsparametem, wie z. B. der Temperatur, der Salzkonzentration und/oder dem pH-Wert, ab, die über einen längeren Zeitraum auf einer Fläche von typischerweise einigen 100 µm² bis zu einigen cm² nur schwer konstant zu halten sind. Um die daraus resultierende eingeschränkte Reproduzierbarkeit der Ergebnisse zu verbessern, werden heute oftmals mehrere gleiche Makromolekülsorten auf dem Microarray an verschiedenen Orten vorgesehen, um so eine statistische Aussagen treffen zu können.

Die Flüssigkeit mit dem zu untersuchenden Makromolekül, z. B. einem Oligonukleotid, kann auch durch Mikrokanäle über eine Festkörperoberfläche geschickt werden, entlang derer sich Makromoleküle verschiedener Art als Fängeroligonukleotide befinden ("Geniom® Technology: From DNA-Chips to DNA-Processors" in "Chips to Hits", 06-09. November 2000, Philadelphia, PA, USA; IBC Conferences Inc.).

Das zu untersuchende Oligonukleotid kommt dabei in die Nähe aller Fängeroligonukleotide entlang des Kanales.

Für einen solchen Stand der Technik ist das Microarray z. B. eine Sandwichstruktur mit externen Pumpen, auf dessen Unterseite sich eine CCD-Kamera befindet. Boden und Deckel sind durchsichtig, damit die Fluoreszenz des markierten Probenoligonukleotids im Durchlicht gemessen werden kann. Die Flüssigkeiten mit den verschiedenen Oligonukleotiden werden durch geschlossene Kanäle auf dem Array gepumpt. Angesichts eines typischen Querschnitts der Kanäle von 100 µm mal 100 µm ist bei den notwendigen Längen der Kanäle ein Druck von mehreren bar erforderlich, wodurch sowohl die Zuführungen als auch der Chip selber teuer werden. Die Pumpen und Ventile, die dies leisten, haben notwendigerweise ein Totvolumen, was zu einem Mehrverbrauch der Reagenzien führt. WO 94/27719 und EP 1 053 784 A2 beschreiben Microarrays, deren einzelne Bindungsstellen im Vergleich zu ihrer Umgebung bevorzugt benetzt werden.

WO 99/43688 beschreibt Nukleinsäuren mit fotoreaktiven Gruppen, die auf Festköperoberflächen gebunden werden.

Aus US 5,874,219 ist es bekannt, Arrays in einzelnen Test-wells zusammenzufassen.

Aufgabe der vorliegenden Erfindung ist es, Verfahren und eine Vorrichtung anzugeben, die es gestatten, bei kleinem Probenvolumen eine möglichst reproduzierbare Analyse durchzuführen.

Diese Aufgabe wird mit einem Analyseverfahren mit den Merkmalen des Anspruches 1 gelöst. Die Aufgabe wird zudem mit einer Analysevorrichtung mit den Merkmalen des Anspruches 10 gelöst. Die Unteransprüche sind auf vorteilhafte Ausgestaltungen gerichtet.

Bei einem Verfahren gemäß Anspruch 1 befindet sich auf einem Microarray eine Vielzahl gegebenenfalls verschiedener erster Makromoleküle in bekannter Anordnung. Das Microarray ist erfingdungsgemäß auf einem Bereich einer Festkörperoberfläche angeordnet, der andere Benetzungseigenschaften aufweist als die umgebende Festkörperoberfläche. Eine Flüssigkeit mit einer Vielzahl zweiter Makromoleküle, die sich - durch die Modulation der Benetzungseigenschaften bewirkt - bevorzugt auf diesem Aufenthaltsbereich aufhält, wird auf den bevorzugten Aufenthaltsbereich der Festkörperoberfläche gebracht. Nach der weitgehenden Entfernung der Flüssigkeit von der Festkörperoberfläche werden ortsaufgelöst die noch auf der Festkörperoberfläche befindlichen zweiten Makromoleküle nachgewiesen. Aus der Lage dieser verbliebenen zweiten Makromoleküle wird festgestellt, welche der ersten Makromoleküle mit Makromolekülen der zweiten Vielzahl eine spezifische Bindung eingegangen sind. Daraus läßt sich die Art bzw. die Arten der in der Flüssigkeit enthaltenen zweiten Makromoleküle feststellen und/oder Information über deren Beschaffenheit erhalten.

Durch die Definition eines bevorzugten Aufenthaltsbereiches kann die Fläche auf dem Festkörper, der von der Flüssigkeit mit der zu analysierenden Makromolekülart bedeckt wird, begrenzt werden. Durch Auswahl einer entsprechenden Geometrie ist es möglich, das Probenvolumen sehr klein zu halten und dennoch zu gewährleisten, daß die zweiten Makromoleküle in der Flüssigkeit mit allen ersten Makromolekülen, die in dem Microarray angeordnet sind, in Verbindung kommen. Durch die Auswahl eines bevorzugten Aufenthaltsbereiches durch Modulation der Benetzungseigenschaften erübrigt es sich, die gesamte Chipoberfläche mit der Flüssigkeit zu überschwemmen.

Aufgrund der unterschiedlichen Benetzungseigenschaften hält sich die Flüssigkeit mit den zu untersuchenden Makromolekülen nur auf dem bevorzugten Aufenthaltsbereich auf. Dazu sind keinerlei Kanäle oder Gräben notwendig, die die Bewegung der Flüssigkeit mit den zu untersuchenden Makromolekülen einschränken oder bremsen würden. Eine schnelle Verteilung auf dem bevorzugten Aufenthaltsbereich ist also möglich. Die Oberflächenspannung der Flüssigkeit bewirkt dabei aufgrund der unterschiedlichen Benetzungseigenschaften, daß die Flüssigkeit den bevorzugten Aufenthaltsbereich ohne Einwirkung einer äußeren Kraft nicht verlassen kann.

Die unterschiedlichen Benetzungseigenschaften können z. B. durch eine entsprechende Beschichtung entweder des bevorzugten Aufenthaltsbereiches oder dessen Umgebung realisiert werden. Zum Beispiel können hydrophile bzw. hydrophobe Bereiche definiert werden. Sind die zu untersuchenden Makromoleküle in wäßriger Lösung enthalten, wird der bevorzugte Aufenthaltsbereich z. B. so gewählt, daß er hydrophiler ist als die umgebende Festkörperoberfläche. Dies kann entweder durch eine hydrophile Beschichtung des bevorzugten Aufenthaltsbereiches oder durch eine hydrophobe Umgebung erreicht werden. Eine hydrophobe Umgebung kann z. B. durch eine silanisierte Oberfläche realisiert werden. Je nach den Benetzungseigenschaften der Flüssigkeit, in der die zu untersuchenden Makromoleküle enthalten sind, kann die den Aufenthaltsbereich umgebende Festkörperoberfläche auch hydrophil, lipophob oder lipophil im Vergleich zur Oberfläche des bevorzugten Aufenthaltsbereiches gewählt werden.

Die Benetzungseigenschaften können weiterhin durch Mikrostrukturierung moduliert werden, wie es beim sogenannten Lotuseffekt der Fall ist, der auf der unterschiedlichen Rauhigkeit der Oberfläche beruht. Die unterschiedliche Rauhigkeit bewirkt dabei unterschiedliche Benetzungseigenschaften. Die Rauhigkeit kann z. B. durch Mikrostrukturierung der entsprechenden Oberflächenbereiche erhalten werden, z. B. durch chemische Behandlung oder lonenbestrahlung.

Die Herstellung von Bereichen unterschiedlicher Benetzungseigenschaften ist dabei Verwendung bereits bekannter lithographischer Verfahren und/oder Beschichtungstechnologien einfach und kostengünstig.

Da die erfindungsgemäße Vorrichtung keine mechanischen Begrenzungen, wie z. B. Kanäle, benötigt, ist es auch möglich, nur Teilbereiche des Microarrays mit Flüssigkeit zu beschicken. Eine solche Anwendung ist vorteilhaft, wenn nur eine spezielle Analyse notwendig ist, die nicht alle auf dem Microarray vorhandenen Makromoleküle einbeziehen muß.

Bei einer Ausgestaltung gemäß Anspruch 2 wird zumindest eine Oberflächenwelle in Richtung der Flüssigkeit geschickt. Durch den Impulsübertrag auf die Flüssigkeit kann eine Verteilung und/oder Durchmischung der Flüssigkeit erreicht werden, so daß die zweiten Makromoleküle effektiv und schnell mit den ersten Makromolekülen in Kontakt kommen können. Ebenso wie bei der ersten Ausgestaltung wird daraufhin die Flüssigkeit aus dem Bereich des Microarrays weitgehend entfernt und ortsaufgelöst nachgewiesen, an welchen Stellen des Microarrays zweite Makromoleküle mit den ersten Makromolekülen eine Bindung eingegangen sind und deswegen auf der Festkörperoberfläche noch vorhanden sind.

Bei dieser Ausgestaltung des Verfahrens werden die Makromoleküle, die in der Flüssigkeit enthalten sind, mit Hilfe der Oberflächenwelle durchmischt und auf der Festkörperoberfläche wirksam verteilt. Auf diese Weise wird die Flüssigkeit quasi umgerührt. Lange Reaktionszeiten, wie sie bei konventionellen Verfahren notwendig sind, können somit reduziert werden, da die Bewegung der Moleküle nicht nur durch Diffusion erfolgt. Zudem kann bei dem erfindungsgemäßen Verfahren gemäß der zweiten Ausgestaltung auf andere Transport- oder Mischvorrichtungen, wie mikromechanische oder piezoelektrisch angetriebene Pumpen, verzichtet werden.

Die Verteilung der Flüssigkeit und das Durchmischen erfolgen praktisch drucklos. Das Mischen der Flüssigkeit durch die Oberflächenwelle erzeugt homogene Reaktionsbedingungen, z. B. bezüglich der Temperatur, des pH-Wertes oder der Salzkonzentration, in dem gesamten von der Oberflächenwelle überstrichenen Bereich.

Die Oberflächenwelle kann mit Hilfe mindestens einer Oberflächenwellenerzeugungseinrichtung generiert werden. Die Oberflächenwellen übertragen einen Impuls auf die Flüssigkeit mit den zu untersuchenden Makromolekülen. Der Impulsübertrag wird entweder durch die mechanische Deformation der Festkörperoberfläche oder durch die Kraftwirkung der sie begleitenden elektrischen Felder auf geladene oder polarisierbare Materie erzielt.

Dabei kann die Stärke der Kraftwirkung auf die Flüssigkeit in einem weiten Bereich über die Amplitude der Oberflächenwelle eingestellt werden. Die Oberflächenwelle kann pulsförmig mit Pulsen verschiedener Länge oder kontinuierlich erzeugt werden. Eine Ansteuerung der Oberflächenwellenerzeugungseinrichtung durch entsprechende Software ist einfach möglich.

Oberflächenwellen lassen sich auf piezoelektrischen Substraten oder Substraten mit piezoelektrischen Bereichen, z. B. piezoelektrischen Beschichtungen, erzeugen. Dabei ist es ausreichend, wenn das Substrat bzw. die entsprechende Beschichtung nur in dem Bereich vorliegt, in dem sich die Oberflächenwellenerzeugungseinrichtung befindet. Die Oberflächenschallwelle breitet sich dann auch außerhalb des piezoelektrischen Bereiches aus.

Zur Erzeugung der Oberflächenwelle wird vorteilhaft ein an sich bekannter Interdigitaltransducer eingesetzt. Ein solcher Interdigitaltransducer hat in einfachster Ausführung zwei Elektroden, die fingerartig ineinander greifen. Durch Anlegen eines hochfrequenten Wechselfeldes, z. B. in der Größenordnung von einigen 100 MHz, wird in einem piezoelektrischen Substrat bzw. in einem piezoelektrischen Bereich des Substrates eine Oberflächenwelle angeregt, deren Wellenlänge sich als Quotient aus der Oberflächenschallgeschwindigkeit und der Frequenz ergibt. Die Ausbreitungsrichtung ist senkrecht zu den ineinander greifenden Fingerelektrodenstrukturen. Mit Hilfe eines solchen Interdigitaltransducers läßt sich auf sehr einfache Weise eine sehr definierte Oberflächenwelle erzeugen. Die Herstellung des Interdigitaltransducers ist mit bekannten lithographischen Verfahren und Beschichtungstechnologien kostengünstig und einfach. Interdigitaltransducer können zudem, z. B. durch Einstrahlung eines elektromagnetischen Wechselfeldes in eine mit dem Interdigitaltransducer verbundene Antenneneinrichtung, drahtlos angesteuert werden.

Ein lateral begrenzter Schallpfad der Oberflächenwelle läßt sich bei einer vorteilhaften Ausgestaltung erreichen, wenn sich der Fingerabstand der Interdigitaltransducer zwischen den Elektroden verändert. Bei gegebener Frequenz wird die Resonanzbedingung, daß die Frequenz gleich dem Quotienten aus der Oberflächenschallgeschwindigkeit und dem Fingerabstand ist, nur in einem lateral räumlich begrenzten Bereich erfüllt. Mit einem solchen sogenannten getaperten Transducer kann also ein ausgewählter Bereich gezielt mit einer Oberflächenwelle beschallt werden.

Die Geometrie des bevorzugten Aufenthaltsbereiches kann an die entsprechende Anwendung angepaßt werden. Zum Beispiel kann der bevorzugte Aufenthaltsbereich mäanderförmig durch das Microarray geführt werden, so daß die einzelnen Positionen des Microarrays sich entlang des bevorzugten Aufenthaltsbereiches aufreihen. Dabei ist sichergestellt, daß alle Analysepunkte mit der Flüssigkeit in Berührung kommen. Ebenso kann eine kreuzweise Anordnung vorgesehen sein, bei der sich die einzelnen Positionen des Microarrays in den Kreuzungsbereichen befinden. Bei einer einfachen Ausgestaltung ist das Microarray auf einem begrenzten Aufenthaltsbereich angeordnet, der die einzelnen Analysepunkte des Microarrays eng umschließt.

Die ortsaufgelöste Messung der nach dem Entfernen der Flüssigkeit auf der Festkörperoberfläche verbliebenen zweiten Makromoleküle, die also mit den ersten Makromolekülen hybridisiert haben, kann mit Hilfe einer Fluoreszenzmessung durchgeführt werden. Dazu müssen die zu analysierenden Makromoleküle mit einem Fluoreszenzfarbstoff markiert sein oder einen fluoreszierenden Bestandteil aufweisen.

Alternativ können zu analysierende Makromoleküle mit einer elektrisch aktiven Funktionsgruppe versehen werden oder eine solche umfassen. Die ortsaufgelöste Messung ist dann als elektrische Messung durchführbar.

Auch die Leitfähigkeit oder dielektrische Eigenschaften der zweiten Makromoleküle können zum Nachweis eingesetzt werden.

Ebenso können die zweiten Makromoleküle radioaktiv markiert sein, um die Lage der verbliebenen zweiten Makromoleküle nach dem weitgehenden Entfernen der Flüssigkeit feststellen zu können.

Eine andere Möglichkeit ergibt sich beim Einsatz sogenannter "Beads". Solche z. B. 10 µm große Masseteilchen werden derart funktionalisiert, daß sie nur an bestimmten Makromolekülen haften. Nachträglich kann dann festgestellt werden, an welchen Stellen des Microarrays sich solche Beads befinden. So läßt sich eine Information über die Beschaffenheit und Art der Makromoleküle in dem Microarray selbst erhalten. Die Beads können z. B. als Massebelag an dem einzelnen Punkt des Microarrays festgestellt werden. Dabei kann z. B. ausgenutzt werden, daß eine Oberflächenschallwelle von einem Massebelag auf der Oberfläche gedämpft wird. Wird z. B. mit einem wie oben beschriebenen getaperten Interdigitaltransducer eine lateral begrenzte Oberflächenschallwelle über die Festkörperoberfläche geschickt, so daß sie nur einen oder wenige Punkte des Microarrays trifft, so kann über die Dämpfung dieser Oberflächenwelle festgestellt werden, ob sich an diesen Stellen eine zusätzliche Masse in Form der Beads befindet oder nicht.

Das erfindungsgemäße Verfahren kann z. B. vorteilhaft zur Analyse von Oligonukleotiden eingesetzt werden. Unterschiedliche Oligonukleotide befinden sich in bekannter Anordnung auf dem Microarray. Die Flüssigkeit mit einem zu analysierenden Oligonukleotid wird auf das Microarray gebracht. Es wird ortsaufgelöst festgestellt, an welcher Stelle Oligonukleotide in der Flüssigkeit mit Oligonukleotiden in dem Microarray spezifisch gebunden bzw. hybridisiert haben. Das Verfahren eignet sich also besonders zum DNA-Screening, wobei die Oligonukleotide z. B. aus kurzen DNA-Strängen (Desoxyribonukleinsäuresträngen) bestehen. Ebenso können jedoch unterschiedliche Proteine, unterschiedliche Antigene oder unterschiedliche Antikörper im Microarray vorgesehen bzw. analysiert werden.

Bei einer vorteilhaften Weiterbildung der erfindungsgemäßen Verfahren wird nach dem Aufbringen der Flüssigkeit auf die Festkörperoberfläche zumindest eine Oberflächenwelle über die Festkörperoberfläche geschickt. Aus der Stärke der Oberflächenwelle, die notwendig ist, die Bindung der Makromoleküle in dem Microarray mit den Makromolekülen in der Flüssigkeit aufzulösen, kann auf die Bindungsstärke geschlossen werden. Dabei kann sowohl die mechanische Deformation der Festkörperoberfläche, als auch die Kraft des die Deformation begleitenden elektrischen Feldes die Bindung lösen.

Mit zwei Oberflächenwellen, deren Schallpfade begrenzte Breite haben und aus verschiedenen Richtungen auf das Microarray treffen, kann ein einzelner Punkt zur Überprüfung der Bindungsstärke auf dem Microarray ausgewählt werden. Die Deformation des Festkörpers im Kreuzungspunkt der Oberflächenwellen ist am größten. Eine sich dort befindende Makromolekülbindung wird einer größeren Belastung ausgesetzt, als Bindungen in der Umgebung, in der sich die Oberflächenwellen nicht kreuzen.

Beispielsweise bei einer Fluoreszenzmessung der zweiten Makromoleküle wird die Oberflächenwellenamplitude erhöht und das Fluoreszenzsignal ortsaufgelöst gemessen. Bricht am Ort der Fluoreszenz die Bindung zwischen den ersten und zweiten Makromolekülen durch die erhöhte Amplitude auf, ändert sich dadurch das Fluoreszenzsignal.

Oberflächenwellen können z. B. auch zum Transport der Flüssigkeitsmenge in den Bereich des Microarrays bzw. zum Entfernen der Flüssigkeit von dem Microarray eingesetzt werden. Z. B. kann mit einer Oberflächenwelle aus einem Reservoir, das sich auf der Festkörperoberfläche befindet und z. B. durch einen Bereich gebildet wird, dessen Benetzungseigenschaften so gewählt sind, daß sich die Flüssigkeit bevorzugt darauf aufhält, mit Hilfe einer Oberflächenwelle die Flüssigkeit in Richtung des Microarrays getrieben werden. Nach Abschluß des Experimentes kann der Bereich des Microarrays mit einer Oberflächenwelle beschallt werden, so daß die Flüssigkeit, getrieben durch den Impulsübertrag der Oberflächenwelle, das Microarray wieder verläßt. Bei einer sehr hohen Intensität der Oberflächenwelle ist es auch möglich, die Flüssigkeit vollständig von der gesamten Festkörperoberfläche zu entfernen.

Die Beschallung der Festkörperoberfläche mit der Oberflächenschallwelle bewirkt bei entsprechender Auswahl der Intensität zudem noch eine Reinigung der überstrichenen Bereiche.

Der Einsatz von Oberflächenwellen zur Durchmischung bzw. Verteilung der Flüssigkeit auf der Festkörperoberfläche vermeidet Totvolumina, die bei konventionellen Pumpenanordnungen entstehen. Es entfallen lange Zuleitungen und Ventile, die "freigepumpt" werden müßten. Mit Oberflächenwellen können auch bei kleinsten Flüssigkeitsmengen Turbulenzen über große Distanzen hinweg erzeugt werden. Auf diese Weise wird die durchschnittliche Geschwindigkeit der zu untersuchenden Makromoleküle erhöht und somit die Reaktionszeit verringert. Oberflächenwellen können also vorteilhaft zur Aufbringung und Durchmischung von Proben-Substanzen eingesetzt werden, um die Reaktionszeit in einem sonst diffusionsbestimmten Prozeß zu beschleunigen.

Mit einer Flüssigkeit mit bekannten zweiten Makromolekülen kann in analoger Weise die Art der ggf. unbekannten ersten Makromoleküle auf dem Microarray bestimmt bzw. charakterisiert werden.

Bei einem erfindungsgemäßen Verfahren zur Herstellung eines Microarrays wird eine Flüssigkeit mit zumindest einer Art Makromolekül auf einen Bereich einer Festkörperoberfläche aufgebracht, dessen Benetzungseigenschaften sich von der umgebenden Festkörperoberfläche derart unterscheiden, daß sich die Flüssigkeit bevorzugt darauf aufhält. Die Makromoleküle sind mit einem lichtaktiven Inhibitor abgeschlossen, der eine Bindung mit der Oberfläche verhindert. Wiederum kann die Flüssigkeit aufgrund der Oberflächenspannung und der Benetzungseigenschaften den bevorzugten Bereich der Oberfläche ohne Einwirkung einer äußeren Kraft nicht verlassen. Die Flüssigkeit ist also bereits lokalisiert, ohne daß die gesamte Festkörperoberfläche mit der Flüssigkeit überschwemmt werden müßte. Auf diese Weise wird die notwendige Menge an Reagenz verringert.

Ein Unterbereich des bevorzugten Aufenthaltsbereiches wird lokal beleuchtet, um den Inhibitor der Makromoleküle in dem beleuchteten Bereich zu lösen. Die Makromoleküle in dem beleuchteten Unterbereich können jetzt gebunden werden und werden an den beleuchteten Stellen im bevorzugten Aufenthaltsbereich gebunden. Man kann auf diese Weise genau festlegen, an welcher Stelle des Aufenthaltsbereiches sich das Makromolekül befinden soll.

Gegebenenfalls werden diese Schritte mit einem oder mehreren unterschiedlichen Arten von Makromolekülen wiederholt, um verschiedene Makromoleküle an definierten und bekannten Stellen des bevorzugten Aufenthaltsbereiches zu immobilisieren. Durch die Festlegung eines bevorzugten Aufenthaltsbereiches durch Auswahl seiner Benetzungseigenschaften werden dabei Kanäle, Kanten, Gräben oder andere mechanische Hemmnisse vermieden. Die Flüssigkeit kann sich vor der Bindung in dem bevorzugten Aufenthaltsbereich frei bewegen.

Zum Flüssigkeitstransport und zur Durchmischung beim Aufbau des Microarrays aus ersten Makromolekülen für die photoinduzierte Synthese, z. B. für die Synthese von DNA, können ebenfalls Oberflächenwellen vorteilhaft eingesetzt werden. Der Flüssigkeitstransport ist nicht mehr diffusionsbestimmt und eine homogene Verteilung der Flüssigkeit gewährleistet. Andere Vorteile ergeben sich in analoger Weise wie beim oben beschriebenen Analyseverfahren.

Ausgestaltungen der erfindungsgemäßen Verfahren werden anhand der anlegenden nicht maßstabsgetreuen Figuren im Detail erläutert. Dabei zeigt:
- Figur 1:: eine schematische Darstellung einer Vorrichtung zur Durchführung eines erfindungsgemäßen Verfahrens,
- Figur 2:: eine weitere Vorrichtung zur Durchführung eines erfindungsgemäßen Verfahrens, in schematischer Darstellung,
- Figur 3:: eine dritte Vorrichtung zur Durchführung eines erfindungsgemäßen Verfahrens, in schematischer Darstellung,
- Figur 4:: eine vierte Vorrichtung zur Durchführung eines erfindungsgemäßen Verfahrens, in schematischer Darstellung, und
- Figur 5a bzw. b:: vergrößerte Ausschnitte der in Figur 1 bzw. Figur 4 gezeigten Vorrichtungen.

Die Darstellung der Figuren ist so zu verstehen, daß sie ggf. nur einen Teil eines größeren Systemes zeigen, in dem sich noch weitere Analyse- bzw. Synthesevorrichtungen der erfindungsgemäßen oder anderer Art befinden.

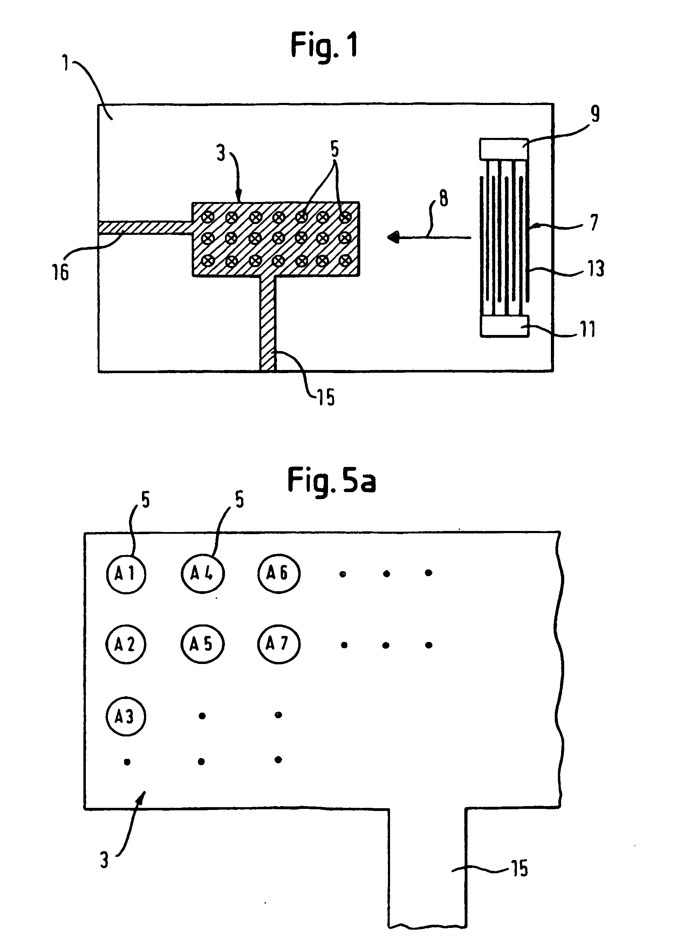

In Figur 1 bezeichnet 1 einen Festkörper, z. B. aus piezoelektrischem Material wie Quarz oder LiNbO₃. Alternativ kann ein Festkörper vorgesehen sein, der zumindest eine teilweise piezoelektrische Oberfläche, z. B. aus ZnO aufweist. 1 kann dabei ein Ausschnitt aus einer größeren Chipeinheit sein.

Auf der Festkörperoberfläche befindet sich ein bevorzugter Aufenthaltsbereich 3, der andere Benetzungseigenschaften als die umgebende Festkörperoberfläche hat. Die Oberfläche des Bereiches 3 ist derart gewählt, daß die Flüssigkeit, in der sich das zu untersuchende Material befindet, bevorzugt darauf aufhält. Bei einer wäßrigen Lösung ist die Oberfläche in dem bevorzugten Oberflächenbereich 3 z. B. hydrophil im Vergleich zu der hydrophoberen Oberfläche des umgebenden Festkörpers gewählt. Dazu kann z. B. die restliche Festkörperoberfläche silanisiert oder mikrostrukturiert und dadurch hydrophob sein.

5 bezeichnet beispielhaft eine Position für eine Art von Makromolekülen, die sich auf dem Aufenthaltsbereich 3 befinden. Es können auch sehr viel mehr Positionen 5 vorgesehen sein. 15 bezeichnet eine Zuführung auf dem Festkörper 1, die dieselben Oberflächeneigenschaften besitzt, wie der bevorzugte Aufenthaltsbereich 3, 16 bezeichnet einen entsprechenden Abfluß. Selbstverständlich können Zu- und Abfluß auch vertauscht sein. 15 und 16 führen in hier nicht weiter ausgeführter Weise z. B. zu Reservoirs oder anderen Analysestationen.

Figur 5a zeigt vergrößert einen Teil des Bereiches 3. Von den Positionen 5 sind nur einige angdeutet.

7 bezeichnet einen Interdigitaltransducer, der als Oberflächenwellenerzeugungseinrichtung dient. Der Interdigitaltransducer 7 besteht aus zwei Elektroden 9 und 11 mit fingerartigen Fortsätzen 13, die ineinander greifen. Bei Anlegen eines Wechselfeldes an die Elektroden des Transducers wird eine Oberflächenwelle mit einer Wellenlänge erzeugt, die dem Fingerabstand der Elektroden entspricht. Die Ausbreitungsrichtung ist senkrecht zu den ineinander greifenden Fingern in Richtung 8. Der Transducer umfaßt eine große Anzahl von ineinander greifenden Fingern, von denen nur einige schematisch und nicht maßstabsgetreu dargestellt sind.

Durch Wahl der Kristallorientierung und/oder der Geometrie der Interdigitaltransducer können dabei verschiedene Wellentypen, wie z. B. Rayleigh-Wellen oder Scherwellen erzeugt werden. Der Interdigitaltransducer 7 wird z. B. mit Hilfe lithographischer Verfahren und Beschichtungsverfahren auf der Chipoberfläche erzeugt.

Selbstverständlich können in nicht gezeigter Weise mehrere Transducer, ggf. mit unterschiedlicher Abstrahlrichtung, um das Microarray angeordnet sein.

Eine solche erfindungsgemäße Vorrichtung kann wie folgt eingesetzt werden. Als Beispiel für die Makromoleküle werden im folgenden Oligonukleotide beschrieben.

Bei dem erfindungsgemäßen Microarray bringt man verschiedenartige DNA-Stränge "A1", "A2", "A3...", sogenannte Oligonukleotide, auf die einzelnen Positionen 5 des Microarrays (siehe z. B. Figur 5a). Die Art des Strangs, gegeben durch die Abfolge der Basen Adenin, Cytosin, Guanin und Thymin, ist dabei bekannt und wird durch die Position in der Matrix gegeben. Typische Abstände zwischen verschiedenartigen Oligonukleotiden betragen dabei etwa 100 µm und die Stränge sind typischerweise 10 bis 100 Basenpaare lang. Die zu identifizierende Oligonukleotidprobe (im folgenden beispielsweise "a1") wird mit einem Fluoreszenzfarbstoff oder auch mit einer elektrisch aktiven Funktionsgruppe markiert und in einer Flüssigkeit gelöst über die Zuführung 15 auf das Array gebracht. Die Benetzungseigenschaften der Zuführung 15 sind so gewählt, daß die Flüssigkeit diesen Bereich 15 nicht seitlich verläßt.

Die Flüssigkeit verteilt sich auf dem bevorzugten Aufenthaltsbereich 3. Nachdem die Flüssigkeit sich auf dem Bereich 3 befindet, wird eine Oberflächenwelle in Richtung 8 mit Hilfe des Interdigitaltransducers 7 erzeugt. Dazu wird an die Elektroden 9, 11 z. B. mit Hilfe von Kontaktdrähten ein Wechselfeld von einigen Megahertz angelegt. Alternativ kann ein Wechselfeld in eine mit den Elektroden verbundene Antennenvorrichtung eingestrahlt werden. Die Oberflächenwelle breitet sich in Richtung 8 aus und fördert durch den Impulsübertrag auf die Flüssigkeit deren Durchmischung und Verteilung auf der bevorzugten Aufenthaltsfläche 3. Auf diese Weise ist sichergestellt, daß die Flüssigkeit sich über den gesamten Bereich 3 bewegt und mit allen Analysepunkten 5 des Microarrays in Verbindung kommt.

Auch für die Bewegung entlang der Zuführung 15 kann eine Oberflächenwelle eingesetzt werden, die von einem auf dem Ausschnitt der Figur 1 nicht sichtbaren Interdigitaltransducer erzeugt wird. In jedem Fall werden die Amplituden der Oberflächenwellen so gewählt, daß die Flüssigkeit den bevorzugten Bereich 3, 15, 16 nicht verläßt.

Ist ein zu der Probe komplementäres "Fänger-Oligonukleotid "A1" auf einer der Positionen 5 in dem Array vorhanden, so kommt es zur Hybridisierung zwischen den komplementären Oligonukleotiden "a1" und "A1".

Im Anschluß wird die Probelösung wiederum durch Einwirkung einer Oberflächenwelle in Richtung 8 durch die Abführung 16 von dem Array gespült. Der elektrisch aktive oder fluoreszierende Marker ist nur noch dort vorhanden, wo das Probenoligonukleotid "a1" mit einem Fängeroligonukleotid hybridisiert hat. Die Fluoreszenz bzw. das elektrische Signal wird nun ortsaufgelöst gemessen. Aus der Lage des Signales wird festgestellt, mit welchem der auf dem Microarray befindlichen DNA-Stränge das Probenoligonukleotid "a1" hybridisiert hat. Somit läßt sich das Probenoligonukleotid "a1" identifizieren.

Durch den begrenzten Aufenthaltsbereich 3 wird das Probenoligonukleotid effektiv an die Fängeroligonukleotide auf den Punkten 5 des Microarrays gebracht. Durch die zusätzliche Durchmischung und Verteilung werden die Reaktionszeiten signifikant verringert.

Die verschiedenartigen DNA-Stränge "A1", "A2", "A3" ... können auf die Matrixform des Microarrays z. B. mit einem Pipettierroboter aufgebracht werden.

Bei einem besonders vorteilhaften Verfahren werden diese sogenannten FängerOligonukleotide auf dem Microarray selbst durch fotoinduzierte Synthetisierung hergestellt. Die einzelnen Basen zur Oligonukleotidsynthetisierung sind dabei jeweils mit einem lichtaktiven Inhibitor abgeschlossen. Das Oligonukleotid kann nur an den Stellen verlängert werden, auf die Licht fällt. Eine gelöste Base, z. B. Guanin, wird auf den bevorzugten Aufenthaltsbereich 3 aufgebracht. Ein Lichtstrahl löst lokal die Inhibitoren an einem Ort 5, an denen das Guanin andocken soll. Nach einer bestimmten Wartezeit hat die Reaktion an den vorbezeichneten Stellen stattgefunden. Diese Reaktion kann ebenfalls durch Mischen mit einer Oberflächenwelle mit Hilfe des Interdigitaltransducers 7 beschleunigt werden. Nach der Reaktion wird die Flüssigkeit mit dem Guanin wieder von dem Array entfernt. Nun kann der gleiche Schritt mit einer anderen Base, z. B. Cytosin, wiederholt werden. Auf diese Weise wird ein Microarray erzeugt, bei dem sich an den einzelnen Stellen verschiedene DNA-Stränge befinden.

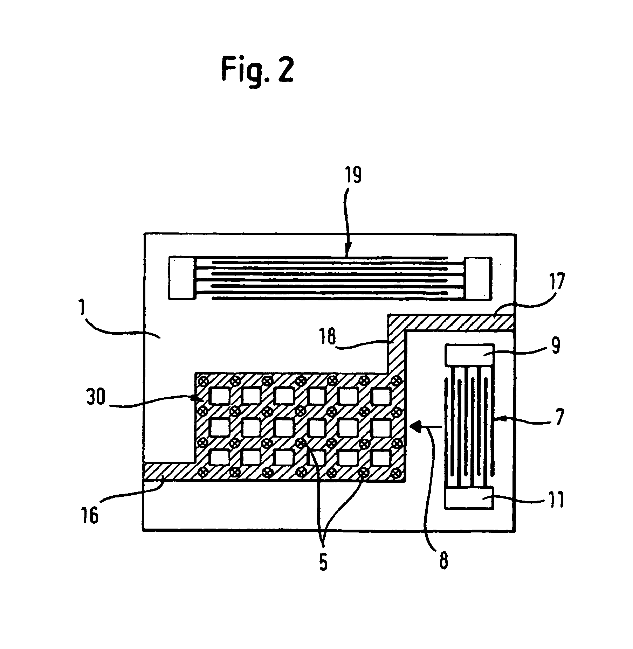

Figur 2 zeigt eine andere Ausführungsform, mit der das erfindungsgemäße Verfahren durchgeführt werden kann. Wiederum ist ein Ausschnitt aus einer Chipoberfläche gezeigt. Hier ist der bevorzugte Aufenthaltsbereich 30 kreuzförmig angeordnet und wiederum schraffiert dargestellt. An den Kreuzungspunkten befinden sich die Analysepunkte 5. Ein zweiter Interdigitaltransducer 19 mit einer Abstrahlrichtung senkrecht zur Abstrahlrichtung 8 des ersten Interdigitaltransducers 7 ist an einer Seite des Microarrays vorgesehen. Mit Hilfe eines solchen zweiten Interdigitaltransducers 19 kann die Durchmischung bzw. Verteilung der Flüssigkeit mit dem Probenoligonukleotid "a1 noch stärker gefördert werden. Selbstverständlich können auch auf den verbleibenden Seiten des Microarrays entsprechende Interdigitaltransducer vorgesehen sein.

Die Flüssigkeit kann auf die Zuführung 17 gebracht werden. Sobald sie den Bereich 18 erreicht hat, kann sie mit Hilfe des Interdigitaltransducers 19, der eine Abstrahlrichtung in Richtung entlang der Zuführung 18 hat, angetrieben werden. Eventuell nachfolgende Flüssigkeit auf dem Bereich 17 wird aufgrund der Oberflächenspannung mitgezogen.

So gelangt die Flüssigkeitsmenge auf den bevorzugten Aufenthaltsbereich 30. Dort kann die Flüssigkeit mit Hilfe der Interdigitaltransducer 7 und 19 effektiv verteilt und durchmischt werden. Der Interdigitaltransducer 7 sorgt für eine Bewegungskomponente in waagerechter Richtung der Figur 2, während der Interdigitaltransducer 19 für eine Bewegungskomponente senkrecht in der Darstellung der Figur 2 sorgt. So läßt sich auf einfache und schnelle Art und Weise eine optimale Verteilung der Flüssigkeit mit einem Probennukleotid auf dem Microarray der Figur 2 erreichen.

Nach Abschluß des Experimentes wird die Flüssigkeitsmenge über die Abführung 16 mit Hilfe einer Oberflächenwelle, die mit dem Interdigitaltransducer 7 erzeugt wird, über die Abführung 16 z. B. in ein nicht gezeigtes Reservoir geführt, das sich ebenfalls auf der Chipoberfläche befinden kann. Die Zu- bzw. Abführungen 16, 17 und 18 in Figur 2 sind ebenso funktionalisiert wie der bevorzugte Aufenthaltsbereich 30.

Analog wie bei der Ausführungsform der Figur 1 kann bei der Ausführungsform der Figur 2 auch die Aufbringung der ersten Makromoleküle durchgeführt werden. Die kreuzweise Anordnung des Aufenthaltsbereiches 30 bei der Ausführungsform der Figur 2 verringert die Flüssigkeitsmenge, die notwendig ist, um das Probennukleotid wirksam in den Bereich aller Fängernukleotide "A1", "A2", "A3" ... zu bringen.

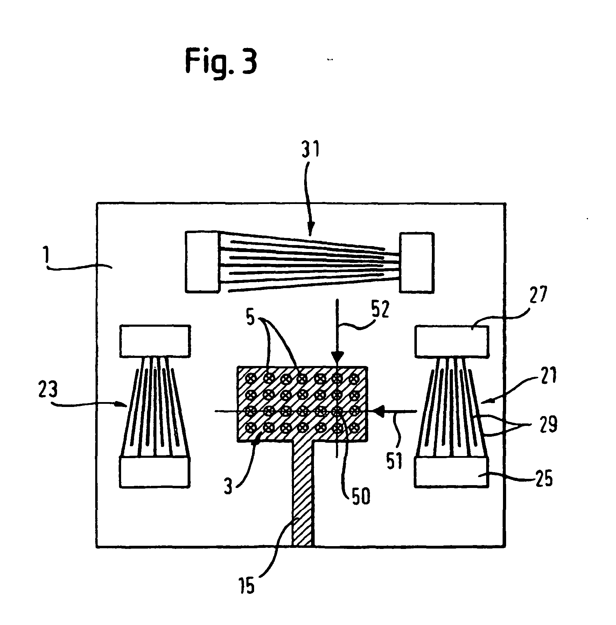

In Figur 3 ist eine andere Ausgestaltung wiederum als Ausschnitt aus einer Chipoberfläche gezeigt. Hier sind die Interdigitaltransducer 21, 23 und 31 als sogenannte "getaperte" Interdigitaltransducer ausgestaltet. Der Interdigitaltransducer 21 umfaßt Elektroden 25 und 27 mit fingerartigen Fortsätzen 29. Der Abstand dieser Finger ist entlang der Verbindungsachse zwischen den Elektroden 25 und 27 nicht konstant. Der Fingerabstand bestimmt die Wellenlänge der abgestrahlten Oberflächenwelle. Bei konstanter Oberflächenwellenschallgeschwindigkeit ist bei einer bestimmten angelegten Frequenz also nur für einen bestimmten Fingerabstand die Resonanzbedingung erfüllt, daß sich die Frequenz der Oberflächenwelle als Quotient aus der Oberflächenschallgeschwindigkeit und der Wellenlänge ergibt. Auf diese Weise läßt sich eine Oberflächenwelle erzeugen, die nur eine sehr geringe seitliche Ausdehnung senkrecht zur Ausbreitungsrichtung und eine definierte Position entlang der Achse des Transducers hat.

Die Interdigitaltransducer 23 und 31 sind analog aufgebaut, wobei der Interdigitaltransducer 23 eine Ausbreitungsrichtung der Oberflächenwelle entgegen der Ausbreitungsrichtung einer Oberflächenwelle des Interdigitaltransducers 21 aufweist. Der Interdigitaltransducer 31 hat eine Ausbreitungsrichtung senkrecht dazu. Auch hier können natürlich zusätzliche Interdigitaltransducer vorgesehen sein, z. B. ein Interdigitaltransducer mit einer Ausbreitungsrichtung entgegen einer Ausbreitungsrichtung des Interdigitaltransducers 31.

Beispielhaft ist eine Oberflächenwelle, die mit dem Interdigitaltransducer 21 erzeugt wird, mit der Ausbreitungsrichtung 51 gezeigt. 52 bezeichnet die Ausbreitungsrichtung einer Oberflächenwelle, die mit dem Interdigitaltransducer 31 erzeugt werden kann. Durch Wahl der Frequenz der an den beiden Interdigitaltransducern anliegenden Oberflächenwelle kann der Analysepunkt 50 ausgewählt werden, an dem die maximale Intensität anliegt.

Eine solche Vorrichtung läßt sich wie folgt vorteilhaft einsetzen.

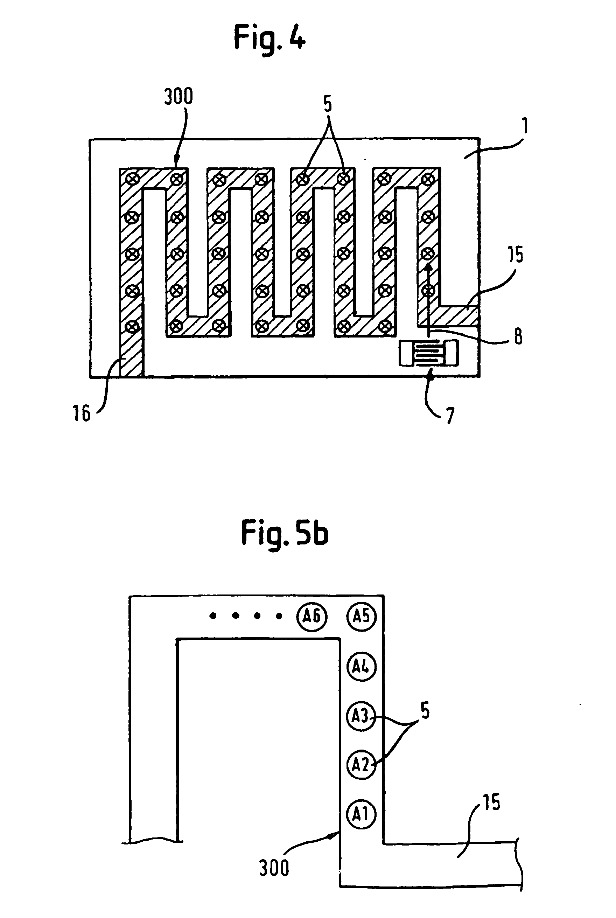

Zunächst wird wiederum eine Flüssigkeit z. B. mit dem Probenoligonukleotid "a1" über die Zuführung 15 auf den bevorzugten Aufenthaltsbereich 3 aufgebracht, auf dem sich verschiedene Fängernukleotide "A1", "A2", "A3" ... wie in Figur 5b angedeutet befinden. Durch Einstrahlen einer Oberflächenwelle wird die Durchmischung und Verteilung der Flüssigkeit mit dem Probenoligonukleotid "a1" auf dem bevorzugten Bereich 3 beschleunigt. Nach der Reaktionszeit wird die Flüssigkeit wiederum von der Oberfläche weitgehend weggewaschen und nur an den Stellen, an denen das Probenoligonukleotid "a1" mit einem Fängeroligonukleotid "A1" auf dem Microarray hybridisiert, verbleibt das Probenoligonukleotid "a1". Die Oberflächenwelle wird dabei entlang der Verbindungslinie zwischen den Elektroden 25 und 27 durch Einsatz verschiedener Frequenzen verschoben.

Eine ortsaufgelöste Messung erlaubt die Feststellung, an welchen der Makromoleküle das Probennukleotid "a1" hybridisiert hat. Dazu kann z. B. wieder eine Fluoreszenzmessung durchgeführt werden, wie oben bereits beschrieben.

Mit Hilfe des getaperten Interdigitaltransducers 21 kann nun eine Oberflächenwelle erzeugt werden, die genau auf den Analysepunkt 50 trifft, an der sich das Oligonukleotid "a1" nach der Hybridisierung befindet. Durch Einstrahlen einer Oberflächenwelle mit entgegengesetzter Ausbreitungsrichtung aber gleicher Frequenz läßt sich mit Hilfe des Interdigitaltransducers 23 an dem Ort des Probenoligonukleotides "a1" ein dynamisches Potential erzeugen. Je stärker die Oberflächenwellen sind, desto stärker ist der Impulsübertrag. Ab einer gewissen Stärke des dynamischen Potentiales wird die Bindung zwischen "a1" und dem korrespondierenden Fängeroligonukleotid "A1" aufgebrochen und das fluoreszierende Oligonukleotid "a1" verläßt den Ort in dem Microarray. Auf diese Weise läßt sich die Bindungsstärke des Probenoligonukleotides "a1" an der entsprechenden Stelle des Microarrays überprüfen. Der Kraftübertrag auf die Bindung kann mit Hilfe der Oberflächenwellen sowohl mechanisch als auch elektrisch durch die die Deformation der Festkörperoberfläche begleitenden elektrischen Felder erzeugt werden. Um einen bestimmten Analysepunkt 50 auszuwählen, können wie oben beschrieben zwei Interdigitaltransducer 21 bzw. 31 mit zueinander senkrecht stehenden Ausbreitungsrichtungen 51 bzw. 52 eingesetzt werden.

Aus der so charakterisierten Bindungsstärke kann man z. B. feststellen, ob das Probenoligonukleotid und das Fängeroligonukleotid 19 komplementäre Basenpaare besitzen oder 20.

Nach Abschluß des Experimentes kann der Interdigitaltransducer 31 zur Erzeugung einer Oberflächenwelle eingesetzt werden, mit der die Flüssigkeit über den Zuleitungs- bzw. Abführungskanal 15 von dem Analysebereich 3 weggetrieben wird.

Bei einer Ausführungsform der Figur 4 ist der Aufenthaltsbereich 300 mäanderförmig gestaltet, wobei sich die Fängeroligonukleotide entlang dieses mäanderförmigen Bereiches aufreihen. Figur 5b zeigt einen Ausschnitt des Bereiches 300. Von den Positionen 5 sind wiederum nur einige beispielhaft gezeigt. Mit Hilfe des Interdigitaltransducers 7 kann die Flüssigkeitsmenge entlang dieses mäanderförmigen Aufenthaltsbereiches getrieben werden, so daß sichergestellt ist, daß die Probennukleotide in der Flüssigkeit in große Nähe zu den Fängeroligonukleotiden kommen. Zur Unterstützung der Bewegung entlang des mäanderförmigen Aufenthaltsbereiches 300 können noch andere, nicht gezeigte Interdigitaltransducer vorgesehen sein.

Selbstverständlich lassen sich die verschiedenen Geometrien, wie sie beispielhaft in den Figuren 1 bis 4 gezeigt sind, auch kombinieren. Schließlich können in jeder der Ausgestaltungen zusätzliche Interdigitaltransducer zur Verstärkung der erfindungsgemäßen Wirkungen eingesetzt werden.

Jede der einzelnen Vorrichtungen kann Teil eines größeren Komplexes auf einer Festkörperoberfläche sein, auf der sich mehrere Analysestationen und/oder Reservoire für Flüssigkeiten befinden, um ein Netzwerk oder ein sogenanntes "Lab on the chip" zu realisieren.

## Patentansprüche

1. Verfahren zur Analyse von Makromolekülen mit Hilfe eines Microarrays, auf dem sich eine Vielzahl von ersten zumindest teilweise unterschiedlichen Makromolekülen in bekannter Anordnung befindet, wobei das Microarray auf einer ebenen Festkörperoberfläche (1) angeordnet ist, auf der ein Bereich (3, 30, 300) definiert ist, dessen Benetzungseigenschaften sich von der umgebenden Festkörperoberfläche derart unterscheiden, daß sich eine Flüssigkeit mit einer Vielzahl von zweiten Makromolekülen bevorzugt darauf aufhält, wobei die Festkörperoberfläche zur Definition dieses Bereichs keine Gräben oder Kanäle aufweist und der Bereich (3, 30, 300) das Microarray umfaßt, bei dem
die Flüssigkeit mit der Vielzahl zweiter Makromoleküle auf den so definierten bevorzugten Aufenthaltsbereich (3, 30, 300) der Festkörperoberfläche gebracht wird, um eine Reaktion der zweiten Makromoleküle mit den ersten Makromolekülen zu ermöglichen,
die Flüssigkeit von dem bevorzugten Aufenthaltsbereich (3, 30, 300) zumindest weitgehend wieder entfernt wird, und
ortsaufgelöst die nach dem Entfernungsprozeß verbliebenen zweiten Makromoleküle nachgewiesen werden, wobei aus der Lage dieser verbliebenen zweiten Makromoleküle festgestellt wird, welche der ersten Makromoleküle mit Makromolekülen der zweiten Vielzahl von Makromolekülen eine Bindung eingegangen sind, um so die Art bzw. die Arten der in der Flüssigkeit enthaltenen zweiten Makromoleküle festzustellen und/ oder Information über deren Beschaffenheit zu erhalten.

2. Verfahren zur Analyse von Makromolekülen nach Anspruch 1, bei dem
zumindest eine Oberflächenwelle in Richtung (8, 51, 52) der Flüssigkeit geschickt wird, die durch Impulsübertrag auf die Flüssigkeit zur gezielten Verteilung auf der Festkörperoberfläche und/oder Durchmischung der Flüssigkeit führt.

3. Verfahren nach einem der Ansprüche 1 oder 2, bei dem die zu analysierenden zweiten Makromoleküle mit einem Fluoreszenzfarbstoff markiert sind oder einen fluoreszierenden Bestandteil aufweisen und die ortsaufgelöste Messung eine Fluoreszenzmessung umfaßt.

4. Verfahren nach einem der Ansprüche 1 bis 3, bei dem die zu analysierenden zweiten Makromoleküle eine elektrisch aktive Funktionsgruppe umfassen oder mit einer solchen markiert sind und die ortsaufgelöste Messung eine elektrische Messung umfaßt.

5. Verfahren nach einem der Ansprüche 1 bis 4, bei dem die zu analysierenden zweiten Makromoleküle zur ortsaufgelösten Messung radioaktiv markiert sind.

6. Verfahren nach einem der Ansprüche 1 bis 5, bei dem die Vielzahl erster Makromoleküle unterschiedliche Oligonukleotide und/oder unterschiedliche Proteine und/oder unterschiedliche Antigene und/oder unterschiedliche Antikörper umfaßt, deren Lage auf dem Microarray bekannt ist.

7. Verfahren nach einem der Ansprüche 1 bis 6, bei dem entweder bevor bzw. nachdem die Flüssigkeit von der Flüssigkeitsoberfläche (1) entfernt wird bzw. wurde, zumindest eine Oberflächenwelle über die Festkörperoberfläche (1) geschickt wird um Information über die Stärke der Bindung des oder der nach dem Entfernungsprozeß verbliebenen zweiten Makromoleküle mit den ersten Makromolekülen zu erhalten.

8. Verfahren nach einem der Ansprüche 1 bis 7, bei dem die Festkörperoberfläche (1) mit einer Oberflächenwelle beschallt wird, um die Flüssigkeit in den Bereich des Microarrays zu bringen und/oder davon zu entfernen.

9. Verwendung eines Verfahrens nach einem der Ansprüche 1 bis 8 zur Analyse von Oligonukleotiden als zweite Makromoleküle.

10. Analysevorrichtung für Makromoleküle zur Durchführung des Verfahrens nach Anspruch 2 bzw. eines Verfahrens nach einem der Ansprüche 3 bis 8, insoweit er direkt oder indirekt von Anspruch 2 abhängig ist, mit einem bevorzugten Aufenthaltsbereich (3, 30, 300) auf einer ebenen Festkörperoberfläche (1), auf der ein Microarray angeordnet ist, wobei der bevorzugte Aufenthaltsbereich (3, 30, 300) andere Benetzungseigenschaften aufweist, als die umgebende Festkörperoberfläche und die Festkörperoberfläche zur Definition des bevorzugten Aufenthaltsbereiches keine Kanäle oder Gräben aufweist, und
zumindest einer Oberflächenwellenerzeugungseinrichtung (7, 19, 21, 23) zur Abstrahlung einer Oberflächenwelle in Richtung (8, 51, 52) des bevorzugten Aufenthaltsbereiches (3, 30, 300).

11. Analysevorrichtung für Makromoleküle nach Anspruch 10, bei der der bevorzugte Aufenthaltsbereich (300) mäanderförmig auf der Festkörperoberfläche (1) angeordnet ist.

12. Analysevorrichtung für Makromoleküle nach Anspruch 10, bei der der bevorzugte Aufenthaltsbereich (30) kreuzförmig auf der Festkörperoberfläche (1) angeordnet ist.

## Claims

1. A method for the analysis of macromolecules using a microarray on which a plurality of first at least partly different macromolecules are located in a known arrangement, wherein the microarray is arranged on a planarsolid body surface (1) on which a region (3, 30, 300) is defined whose wetting properties differ from the surrounding solid body surface such that a liquid with a plurality of second macromolecules preferably resides thereon, with the solid body surface having no trenches or channels for the definition of this region and the region (3, 30, 300) including the microarray, wherein
the liquid with the plurality of second macromolecules is brought onto the so detected preferred residence region (3, 30, 300) of the solid body surface in order to make possible a reaction of the second macromolecules with the first macromolecules,
the liquid is at least largely removed again from the preferred residence region (3, 30, 300), and
the second macromolecules remaining after the removal process are detected in a spatially resolved fashion wherein it is established from the position of these remaining second macromolecules which of the first macromolecules have formed a bond with macromolecules of the second plurality of macromolecules in order to thus determine the type or types of second macromolecules contained in the liquid and/or to obtain information on their character.

2. A method for the analysis of macromolecules according to claim 1, wherein at least one surface wave is launched in the direction (8, 51, 52) of the liquid which leads to the specific distribution over the solid body surface and/or to thorough mixing of the liquid by pulse transmission to the liquid.

3. A method according to any one of claims 1 or 2, wherein the second macromolecules to be analysed are labelled with a fluorescent dye or have a fluorescent component and the spatially resolved measurement comprises a fluorescence measurement.

4. A method according to any one of claims 1 to 3, wherein the second macromolecules to be analysed comprise an electrically active function group or are labelled with one such group and the spatially resolved measurement comprises an electrical measurement.

5. A method according to any one of claims 1 to 4, wherein the second macromolecules to be analysed are radioactively labelled for spatially resolved measurement.

6. A method according to any one of claims 1 to 5, wherein the plurality of first macromolecules comprises different oligonucleotides and/or different proteins and/or different antigens and/or different antibodies whose position on the microarray is known.

7. A method according to any one of claims 1 to 6, wherein either before or after the liquid is or has been removed from the liquid surface (1), at least one surface wave is launched over the solid body surface (1) in order to obtain information on the strength of the bond between the second macromolecule(s) remaining after the removal process and the first macromolecules.

8. A method according to any one of claims 1 to 7, wherein the solid body surface (1) is acoustically irradiated by a surface wave in order to bring the liquid into the region of the microarray and/or remove it therefrom.

9. Use of a method according to any one of claims 1 to 8 for the analysis of oligonucleotides as second macromolecules.

10. An analytical device for macromolecules for implementing the method according to claim 2 or a method according to any one of claims 3 to 8, insofar as this is directly or indirectly dependent on claim 2, with a preferred residence region (3, 30, 300) being arranged on a planar solid body surface (1), with the preferred residence region (3, 30, 300) having different wetting properties to the surrounding solid body surface and the solid body surface having no trenches or channels for the definition of this region, and
at least one surface wave generating device (7, 19, 21, 23) for radiating a surface wave in the direction (8, 51, 52) of the preferred residence region (3, 30, 300).

11. An analytical device for macromolecules according to claim 10, wherein the preferred residence region (300) is arranged in an meander-shaped fashion on the solid body surface (1).

12. An analytical device for macromolecules according to claim 10, wherein the preferred residence region (30) is arranged in a cross-shaped fashion on the solid body surface (1).

## Revendications

1. Procédé d'analyse de macromolécules à l'aide d'un microarray sur lequel une multitude de premières macromolécules tout au moins partiellement différentes se trouvent dans un agencement connu, le microarray étant agencé sur une surface de corps solide (1) plane sur laquelle est définie une zone (3, 30, 300) dont les propriétés de mouillage se distinguent de la surface de corps solide environnante de telle sorte que de préférence un liquide avec une multitude de deuxièmes macromolécules reste sur celle-ci, la surface des corps solides ne présentant ni fossés ni canaux pour définir cette zone, et la zone (3, 30, 300) comprenant le microarray, dans lequel
le liquide avec la pluralité de deuxièmes macromolécules est amené sur la zone de séjour (3, 30, 300) préférée ainsi que définie de la surface de corps solide pour permettre une réaction des deuxièmes macromolécules avec les premières macromolécules,
le liquide est de nouveau enlevé de la zone de séjour préférée (3, 30, 300) au moins en grande partie, et
les deuxièmes macromolécules restantes après le processus d'enlèvement sont décelées avec résolution locale, et à partir de la position de ces deuxièmes macromolécules restantes, on constate lesquelles des premières macromolécules se sont liées aux macromolécules de la deuxième pluralité de macromolécules pour constater ainsi la nature ou les natures des deuxièmes macromolécules contenues dans le liquide et/ou pour obtenir une information sur leurs propriétés.

2. Procédé pour l'analyse de macromolécules selon la revendication 1, dans lequel
au moins une onde de surface est envoyée en direction (8, 51, 52) du liquide qui, par transmission d'impulsions sur le liquide entraîne la répartition ciblée sur la surface des corps solides et/ou le mélange du liquide.

3. Procédé selon l'une des revendications 1 ou 2, dans lequel les deuxièmes macromolécules à analyser sont marquées avec un colorant fluorescent ou présentent un composant fluorescent et la mesure avec résolution locale comprend une mesure de fluorescence.

4. Procédé selon l'une des revendications 1 à 3, dans lequel les deuxièmes macromolécules à analyser comprennent un groupe fonctionnel électriquement actif ou sont marquées avec un tel groupe et la mesure avec résolution locale comprend une mesure électrique.

5. Procédé selon l'une des revendications 1 à 4, dans lequel les deuxièmes macromolécules à analyser sont marquées radioactivement pour la mesure avec résolution locale.

6. Procédé selon l'une des revendications 1 à 5, dans lequel la pluralité de premières molécules comprennent différentes oligonucléotides et/ou différentes protéines et/ou différents antigènes et/ou différents anticorps, dont la position sur le microarray est connue.

7. Procédé selon l'une des revendications 1 à 6, dans lequel soit avant soit après que le liquide ait ou eût été enlevé de la surface de liquide (1), au moins une onde de surface est envoyée via la surface de corps solide (1) pour obtenir une information sur la force de la liaison entre la ou les deuxièmes macromolécules restantes après le processus d'enlèvement et les premières macromolécules.

8. Procédé selon l'une des revendications 1 à 7, dans lequel la surface de corps solide (1) est attaquée avec une onde surface pour amener le liquide dans la zone du microarray et/ou pour l'enlever de celle-ci.

9. Utilisation d'un procédé selon l'une des revendications 1 à 8 pour l'analyse d'oligonucléotides en tant que deuxièmes macromolécules.

10. Dispositif d'analyse pour macromolécules pour mettre en oeuvre le procédé selon la revendication 2 ou un procédé selon l'une des revendications 3 à 8, dans la mesure où elle dépend directement ou indirectement de la revendication 2, comportant une zone de séjour (3, 30, 300) préférée sur une surface de corps solide (1) plane sur laquelle est agencé un microarray, la zone de séjour (3, 30, 300) préférée présentant d'autres propriétés de mouillage que la surface de corps solide environnante, et la surface de corps solide ne présentant ni canaux ni fossés pour définir la zone de séjour préférée, et
comportant au moins un dispositif de génération d'ondes de surface (7, 19, 21, 23) pour dissiper une onde de surface en direction (8, 51, 52) de la zone de séjour (3, 30, 300) préférée.

11. Dispositif d'analyse pour macromolécules selon la revendication 10, dans lequel la zone de séjour (300) préférée est agencée en forme de méandres sur la surface de corps solide (1).

12. Dispositif d'analyse pour macromolécules selon la revendication 10, dans lequel la zone de séjour (300) préférée est agencée en forme de croix sur la surface de corps solide (1).
